# EUROPÄISCHE PATENTSCHRIFT

(11) **EP 3 209 264 B1**
(45) Veröffentlichungstag und Bekanntmachung des Hinweises auf die Patenterteilung: **17.10.2018**
(21) Anmeldenummer: 15748191.2
(22) Anmeldetag: 22.07.2015
(51) Int. Cl.: A61J 15/00, A61M 39/02, A61M 39/22, A61M 39/10

(54) **PEG-SONDE MIT GESCHÜTZTER MANUELLER VENTILBEDIENUNG**
PEG-TUBE WITH PROTECTED MANUAL VALVE OPERATION
SONDE PEG AVEC OPERATION DE SOUPAPE MANUELLE PROTÉGÉE

(30) Priorität: 21.10.2014 DE 102014115340
(43) Veröffentlichungstag der Anmeldung: 30.08.2017
(73) Patentinhaber: Kliniken der Stadt Köln gGmbH, 51067 Cologne (DE)
(72) Erfinder: DORMANN, Arno, 51069 Köln (DE)
(74) Vertreter: Patentanwälte Bauer Vorberg Kayser
(86) Internationale Anmeldenummer: PCT/EP2015/066781
(87) Internationale Veröffentlichungsnummer: WO 2016/062418

(56) Entgegenhaltungen:
- EP-A1- 2 221 084
- WO-A1-2006/036843
- JP-A- 2003 038 655
- US-A- 5 098 378
- US-A- 5 403 290
- US-A1- 2008 033 364
- US-A1- 2009 318 854

## Beschreibung

Die Erfindung bezieht sich auf eine PEG-Sonde
- mit einer inneren Halteplatte,
- mit einem PEG-Schlauch, der einen inneren Endbereich und einen äußeren Endbereich aufweist, wobei der innere Endbereich mit der inneren Halteplatte fest verbunden ist und dort eine innere Öffnung des PEG-Schlauchs aufweist,
- mit einer äußeren Halteplatte, durch die der PEG-Schlauch geführt ist,
- mit einem Trichteradapter, der dem äußeren Endbereich zugeordnet ist,
- mit einem dicht schließenden Ventil, das am äußeren Endbereich des PEG-Schlauchs angeordnet ist, wobei das Ventil in einer Schließstellung den Durchfluss durch den PEG-Schlauch sperrt und in einer Öffnungsstellung freigibt, wobei das Ventil einen Ventilkörper und ein Ventilgehäuse aufweist, der Ventilkörper mit einer von außen zugänglichen Handhabe verbunden und zwischen der dauerhaften Schließstellung und der dauerhaften Öffnungsstellung bewegbar in dem Ventilgehäuse gelagert ist.

Die Erfindung ist definiert durch den Gegenstand von Anspruch 1. Eine PEG-Sonde ist aus WO 2006/036843 A1 vorbekannt. Eine weitere Sonde ist aus US 4,666,433 A bekannt. Gastronomie-Ernährungssonden bieten Zugang zum Magen am Ort eines Stoma. Derartige Anschlüsse bleiben typischerweise über längere Zeiträume in der Position und dienen zum Ernähren des Patienten und zur Verabreichung von Medikamenten. Mehrere der bekannten PEG-Sonden weisen Rückschlagventile auf, die einen Rückfluss des Mageninhalts durch den PEG-Schlauch und Austritt aus dessen äußerer Öffnung verhindern, siehe beispielsweise US 2006/0079850 A1 und DE 101 54 864 A1. So kann verhindert werden, dass möglicherweise saurer Mageninhalt nach außen austreten und dort Schädigungen hervorrufen kann.

Bei der vorbekannten PEG-Sonde der eingangs genannten Art ist der Trichteradapter mit dem äußeren Endbereich des PEG-Schlauchs fest verbunden und ist ein Verschlussstopfen vorgesehen, mit dem das äußere Ende des Trichteradapters verschlossen werden kann. Derartige Verschlusskappen sind bei PEG-Sonden häufig anzutreffen. Sie dichten den PEG-Schlauch ab, sie vermeiden den Zutritt von Fremdsubstanzen von außen in den PEG-Schlauch. Eine dauerhafte Abdichtung bieten sie aber nicht.

Auch bekannt sind lösbare Klemmverschlüsse, die allgemein auch als Ritsch-Ratsch-Klemme bezeichnet werden und die an beliebiger Stelle des PEG-Schlauchs zwischen der äußeren Halteplatte und dem äußeren Endbereich angebracht werden können. Eine derartige Schlauchklemme ist in DE 100 51 593 A1 allgemein angesprochen. Derartige Schlauchklemmen haben aber den Nachteil, dass sie auf Dauer den PEG-Schlauch stark überbeanspruchen und zerstören. Er wird beim Zusammenquetschen in den dann gebildeten, scharf gebogenen Eckbereichen des Schlauchs übermäßig beansprucht und neigt zur Rissbildung und damit auf Dauer zu einer Undichtigkeit.

Hiervon ausgehend ist es Aufgabe der Erfindung, die PEG-Sonde der eingangs genannten Art dahingehend weiterzubilden, dass ein sicherer Verschluss des PEG-Schlauchs erreicht wird, der beliebig oft geöffnet und geschlossen werden kann, in einer teilweise geöffneten Position dauerhaft gehalten werden kann, der so ausgelegt ist, dass er nur mit Hilfsmitteln und deshalb nicht von einem Patienten einfach und unmittelbar bedienbar ist und der Voraussetzungen dafür bietet, die außerhalb des Körpers befindlichen Teile der PEG-Sonde so auszubilden, dass sie möglichst klein und einfach zu tragen sind.

Diese Aufgabe wird dadurch gelöst durch die Merkmale des Patentanspruchs 1.

Das Ventil ist unmittelbar am äußeren Endbereich des PEG-Schlauchs angeordnet. Dieses Ventil lässt sich In beliebigen Zwischenstellungen zwischen einer Schließstellung und einer Öffnungsstellung einstellen. Alle End- und Zwischenstellungen sind dauerhaft einstellbar. Zumindest in der Öffnungsstellung hat das Ventil einen freien Durchflussquerschnitt, der demjenigen des PEG-Schlauchs entspricht oder größer ist. Das Ventil hat eine Handhabe, durch die Handhabe wird der Ventilkörper bewegt, er kann so zwischen seinen beiden Endstellungen hin- und her bewegt werden. Die Bewegung kann eine Schiebebewegung entlang der Längsachse des PEG-Schlauchs, eine Drehbewegung um die Längsachse des PEG-Schlauchs als Achse und eine Drehbewegung quer zur Längsachse und um eine Achse des Ventilkörpers sein. Im letzteren Fall stellt sich der Vorteil ein, dass lediglich eine Drehbewegung notwendig ist, um den Ventilkörper zu bewegen. Dadurch ist die Abdichtung von innen nach außen einfach.

Der PEG-Schlauch befindet sich nur auf einer Seite des Ventils, der Trichteradapter ist lösbar mit dem Ventil verbunden. An dem vom PEG-Schlauch abgewandten Ende des Ventils wird der Trichteradapter lösbar angesetzt und entnommen. Es ist am Ventil ein Kupplungsstück vorgesehen, der Trichteradapter hat ein Kupplungsgegenstück. Beide Kupplungsstücke können miteinander dicht verbunden werden und bilden dann eine mechanisch feste Einheit. Auf diese Weise muss der Trichteradapter, der im Laufe der Zeit durch verschiedentliche Entwicklungen und auch durch eine zu erwartende Standardisierung immer größeres Volumen hat, nicht ständig mitgeführt werden. Er ist auch einfach gegen ein anderes Modell, mit dem z.B. Medikamente besser verabreicht werden können, austauschbar.

Das Ventil lässt sich günstig reinigen, weil sein Gehäuse aus mindestens zwei Teilstücken gefertigt ist. Sind diese voneinander getrennt, ist der Ventilkörper frei, kann also gereinigt werden. Dies gilt auch für die Teile des Ventilkörpers. Im zusammengefügten Zustand der beiden Teilstücke halten diese den Ventilkörper zwischen sich fest, vorzugsweise elastisch.

Die Handhabe ist mehrteilig ausgebildet. Dabei ist ein erstes Teil permanent am Ventilkörper angeordnet. Mit diesem ersten Teil kann aber ohne Zuhilfenahme eines Werkzeugs der Ventilkörper nicht bewegt werden. Erst das zweite Teil bringt das notwendige Hilfsmittel bzw. Werkzeug, das erste Teil mit daran befindlichem Ventilkörper bewegen zu können. Das zweite Teil ist es so am Trichteradapter angeordnet, dass nur bei am Ventil angesetztem Trichteradapter ein Betätigen des Ventilkörpers möglich ist. Die Verbindung zwischen Trichteradapter und Ventil ist so ausgebildet, dass der Trichteradapter nur dann vom Ventil gelöst werden kann, wenn sich das Ventil in der Schließstellung befindet. Dadurch ist sichergestellt, dass bei Abziehen des Trichteradapters das Ventil geschlossen ist. Ohne Trichteradapter kann ein Patient ohne Hilfsmittel nicht einfach das Ventil öffnen.

Beim Zusammenstecken von Ventil und Trichteradapter greift das zweite Teil der Handhabe, das mit dem Trichteradapter zusammenhängt, selbsttätig in das erste Teil ein oder kuppelt anderweitig mit ihm, so dass mittels des zweiten Teils das erste Teil bedient werden kann. Das zweite Teil weist ein Werkzeug auf, das erste Teil hat ein dazu passendes Gegenwerkzeug. Wenn beide im Eingriff miteinander sind, ist eine Betätigung des Ventilkörpers mit einfachen Mitteln manuell möglich. Hierzu wird ein Griffteil des zweiten Teils manuell bewegt, z.B. gedreht. Ohne das zweite Teil kann der Ventilkörper nur mit Spezialwerkzeug betätigt werden, jedenfalls nicht mit manuellen Mitteln, beispielsweise nicht mit den Fingern eines Patienten.

Vorzugsweise wird das Werkzeug, das sich am zweiten Teil der Handhabe befindet, beim Zusammenstecken von Trichteradapter und Ventil in einer Rille geführt. Dadurch ist eine gewisse Orientierung von Trichteradapter und Ventil zueinander erzwungen, beide können nur dann zusammengesteckt werden, wenn das Werkzeug in die Rille passt. Die Rille führt das Werkzeug beim Zusammenschieben dergestalt, dass das Werkzeug in das Gegenwerkzeug des ersten Teils eingreift oder anderweitig kuppelt. Anstelle einer Rille kann auch eine Rippe vorgesehen sein. Es ist möglich, die Rille mit einer Schräge auszubilden, so dass das Werkzeug in radialer Richtung angehoben wird, wenn Trichteradapter und Ventil zusammengefügt werden. Dadurch ist das Werkzeug angehoben und kann in das Gegenwerkzeug einklicken bzw. einrasten, sobald die Endposition erreicht ist.

In einer anderen Ausbildung ist das zweite Teil der Handhabe flexibel mit dem Trichteradapter verbunden, z.B. über eine Schnur oder eine biegsame Lasche. Das zweite Teil steht zur Verfügung, wenn der Trichteradapter in der Nähe ist, insbesondere, wenn er mit dem Ventil verbunden ist.

Vorzugsweise ist der Ventilkörper ein geometrisch einfacher Gegenstand. Es bieten sich kugelförmige Ventilkörper, kegelförmige oder zylindrische Ventilkörper und scheibenförmige Ventilkörper an.

Das Ventil ist auf jeden Fall auf einer Seite mit dem PEG-Schlauch verbunden. Für die Verbindung von Ventil und PEG-Schlauch können Klemmverschraubungen eingesetzt werden, wie sie im Stand der Technik bekannt sind, siehe beispielsweise US 2006/0079850 A1, die jedoch keine klemmende Überwurfmutter zeigt.

Weitere Vorteile und Merkmale der Erfindung ergeben sich aus den übrigen Ansprüchen sowie der nun folgenden Beschreibung von nicht einschränkend zu verstehenden Ausführungsbeispielen der Erfindung, die im Folgenden unter Zuhilfenahme der Zeichnung näher erläutert werden. In dieser Zeichnung zeigen:
- Fig. 1:: eine perspektivische Darstellung der PEG-Sonde nach einem ersten Ausführungsbeispiel, das nicht dem Wortlaut des Anspruchs 1 entspricht,
- Fig. 2:: ein axiales Schnittbild durch ein Ventil mit einem zweiteiligen Ventilgehäuse,
- Fig. 3:: eine Draufsicht auf ein Ventil, das mit einem Trichteradapter kuppelbar ist,
- Fig. 4:: eine Draufsicht auf einen Trichteradapter, der passend zum Ventil nach Figur 3 ist und mit diesem verbindbar ist,
- Fig. 5:: einen Schnitt durch das Ventil nach Figur 3 entlang der Schnittlinie V-V,
- Fig. 6:: ein Schnittbild durch den Trichteradapter nach Fig. 4 entlang der Schnittlinie VI-VI in Figur 4 und
- Fig. 7:: eine prinzipielle Darstellung eines Ventils und eines mit diesem kuppelbaren Trichteradapters, am Trichteradapter ist eine Lasche vorgesehen, an der ein zweites Teil der Handhabe flexibel angeordnet ist.

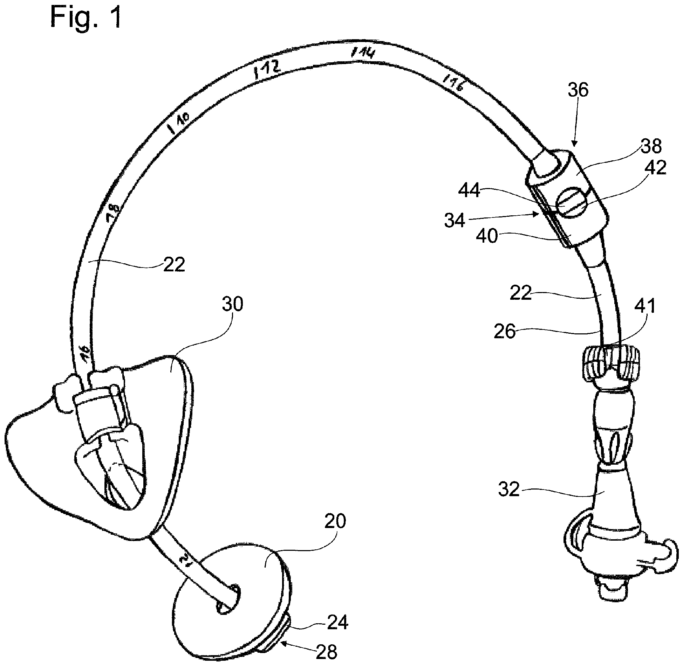

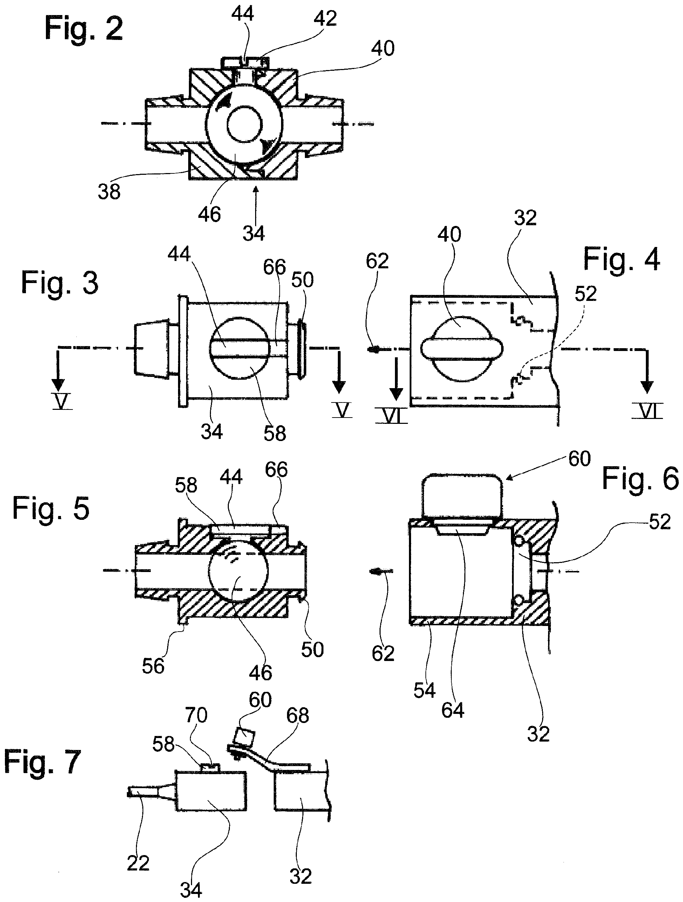

Figur 1 zeigt ein erstes Ausführungsbeispiel, das nicht dem Wortlaut des Anspruchs 1 entspricht. Figur 2 zeigt ein zweites Ausführungsbeispiel, die Figuren 3 bis 6 zeigen ein drittes Ausführungsbeispiel, ein viertes Ausführungsbeispiel ist in Figur 7 gezeigt. Einzig das erste Ausführungsbeispiel zeigt eine komplette PEG-Sonde, die sonstigen Ausführungsbeispiele zeigen Teile.

Aus Figur 1 ist eine PEG-Sonde mit einer inneren Halteplatte 20, auch "bumper" genannt, ersichtlich. Mit ihr ist ein PEG-Schlauch 22 fest verbunden. Der PEG-Schlauch 22 hat einen inneren Endbereich 24 und einen äußeren Endbereich 26. Der innere Endbereich 24 ist mit der inneren Halteplatte 20 fest verbunden und hat eine innere Öffnung 28. Aus dieser kann eine Substanz, beispielsweise zum Zwecke des Ernährens oder der medikamentösen Behandlung, in einen Magen (nicht dargestellt, siehe US 4,666,433 A, Fig. 3) eingeleitet werden. Als PEG-Schlauch 22 wird ein Schlauch nach dem Stand der Technik verwendet. Er ist vorzugsweise glasklar, biegsam und hat einen freien Innenquerschnitt von 2 bis 5 mm, der Querschnitt ist rund.

Weiterhin hat die PEG-Sonde eine äußere Halteplatte 30. Durch sie ist der PEG-Schlauch 22 hindurchgeführt. Er ist in der äußeren Halteplatte 30 fixiert, er kann dabei mittels einer Klemmvorrichtung der äußeren Halteplatte 30 an beliebigen Orten positioniert werden. Dies ist Stand der Technik. Auf diese Weise kann der Abstand zwischen der inneren Halteplatte 20 und der äußeren Halteplatte 30 auf den jeweiligen Patienten eingestellt werden.

Schließlich hat die PEG-Sonde am äußeren Endbereich 26 einen Trichteradapter 32. Im gezeigten Ausführungsbeispiel ist er über eine Klemmverschraubung mit dem äußeren Endbereich 26 verbunden. Der gezeigte Trichteradapter 32 ist Stand der Technik. Er hat eine Verschlusskappe.

Im PEG-Schlauch 22 befindet sich ein Ventil 34, es ist zwischen der äußeren Halteplatte 30 und dem äußeren Endbereich 26 angeordnet. Dabei befindet sich auf beiden Seiten des Ventils 34 ein Stück des PEG-Schlauchs 22. Das Ventil 34 ist beispielsweise über eine Klemmverschraubung beidseitig jeweils mit dem angrenzenden Stück PEG-Schlauch 22 verbunden, wie sie für den Trichteradapter 32 benutzt wird.

Das Ventil 34 hat ein zweiteiliges Ventilgehäuse 36, nämlich ein erstes Teilstück 38 und ein zweites Teilstück 40. Weiterhin hat es eine Handhabe 42, die im gezeigten Ausführungsbeispiel als kreisrunde Scheibe mit einem Betätigungsschlitz 44 ausgebildet ist. Sie befindet sich zwischen den beiden Teilstücken 38, 40.

Figur 2 zeigt im Schnitt ein Ventil 34, wie es bei der PEG-Sonde nach Figur 1 eingesetzt werden kann. Das Ventil 34 hat wiederum ein zweiteiliges Ventilgehäuse 36 mit einem ersten Teilstück 38 und einem zweiten Teilstück 40, die im gezeigten Zustand dicht miteinander verbunden sind, sie sind mechanisch miteinander verbunden. Dies geschieht über geeignete Mittel, wie sie aus dem Stand der Technik bekannt sind, beispielsweise eine Steck- oder Rastverbindung. Jedes Teilstück 38, 40 hat an seiner Außenseite einen kegelförmigen Bereich, dort kann der PEG-Schlauch 28 aufgeschoben werden. Er wird mittels geeigneter Vorrichtungen, wie sie beispielsweise am Trichteradapter 32 ausgebildet sind, festgehalten. Das geschieht insbesondere mittels einer Überwurfmutter 41.

Zwischen sich definieren die beiden Teilstücke 38, 40 einen kugelförmigen Hohlraum, der von einem Ventilkörper 46 in Form einer Kugel mit einer diametralen Bohrung 48 ausgefüllt ist. Dieser Ventilkörper 46 ist mit einem kurzen zylindrischen Abschnitt verbunden, der radial verläuft und Teil einer Handhabe 42 ist, die weiterhin eine außerhalb der beiden Teilstücke 38, 40 befindliche Scheibe aufweist, in der ein Betätigungsschlitz 44 vorgesehen ist. Das Ventil 34 nach Figur 2 ist im Sperrzustand gezeigt. Es ist zu erkennen, dass der Durchmesser der Bohrung 48 dem Durchmesser von Passagen in den beiden Teilstücken 38, 40 entspricht. Wird der Ventilkörper 46 um 90° um eine in der Papierebene liegende Achse mittels der Handhabe 42 geschwenkt, wird ein durchgehender Kanal gleichbleibenden Querschnitts gebildet.

Das Ventil nach Figur 2 kann gereinigt werden, in dem die beiden Teilstücke 38, 40 voneinander separiert werden. Dann ist der Ventilkörper 46 zusammen mit der mit ihm verbundenen Handhabe 42 frei, er kann ebenso wie die Teilstücke 38, 40 gereinigt werden.

Beim dritten Ausführungsbeispiel nach den Figuren 3 bis 6 und auch bei dem vierten Ausführungsbeispiel nach Figur 7 befindet sich der PEG-Schlauch 22 nur auf einer, in der Zeichnung linken Seite des Ventils 34. Auf der anderen Seite des Ventils 34 ist jeweils ein Kupplungsstück 50 vorgesehen. Es wirkt mit einem Kupplungsgegenstück 52 zusammen, das am Trichteradapter 32 ausgebildet ist. Beim dritten Ausführungsbeispiel bildet der Trichteradapter bildet eine Hülse aus, die im zusammengesteckten Zustand von Ventil 34 und Trichteradapter 32 den zylindrischen Außenmantel des Ventils 34 übergreift. Es ist ein Kragen 56 vorgesehen, der ringsum radial am Außenmantel vorspringt und sich in Nähe einer freien Vorderkante der Hülse 54 befindet, wenn Ventil 34 und Trichteradapter 32 zusammengesteckt sind. Ein Kragen kann entfallen. Dies insbesondere, wenn die Hülse 54 in ihrem freien Innenraum so lang gemacht wird, dass sie das Ventilgehäuse 36 vollständig übergreift.

Der Ventilkörper 46 wird wiederum durch eine Kugel gebildet. Sie ist ähnlich wie im zweiten Ausführungsbeispiel nach Figur 2 mit einer Handhabe 42 verbunden.

Im dritten und vierten Ausführungsbeispiel ist die Handhabe 42 zweiteilig ausgebildet. Ein erstes Teil 58 der Handhabe 42 ist ebenso wie im zweiten Ausführungsbeispiel permanent mit dem Ventilkörper 46 verbunden. Dieses erste Teil 58 genügt für die Betätigung des Ventilkörpers 46, wenn man ein geeignetes Werkzeug hat. Dieses geeignete Werkzeug wird durch ein zweites Teil 60 der Handhabe 42 gebildet. Prinzipiell könnte aber das Ventil 34 bereits auch ohne dieses zweite Teil betätigt werden. Die Absicht ist, dass das erste Teil einen Betätigungsschlitz 44 oder eine Vertiefung, einen Vorsprung oder ein Lochmuster, also eine Verschlüsselung für die Betätigung hat, wodurch es erforderlich ist, dass man nur mit einem passenden, speziellen Werkzeug das erste Teil 58 bewegen kann. Auf keinen Fall soll das erste Teil 58 lediglich mit den Fingern betätigt werden können. Die Verschlüsselung und auch die Kraftanstrengung, die für das Betätigen des Ventilkörpers 46 erforderlich sind, sind so abgestimmt, dass ein Patient nicht mit normalerweise ihm zur Verfügung stehenden Mitteln das Ventil 34 betätigen kann.

Manuell und ohne irgendwelche fremden Werkzeuge kann das Ventil 34 betätigt werden, wenn der zweite Teil 60 mit dem ersten Teil 58 zusammenwirkt. Dies erfolgt dann, wenn im dritten Ausführungsbeispiel Trichteradapter 32 und Ventil 34 im Sinne des Pfeils 62 bis zum Anschlag zusammengeschoben werden. Das zweite Teil 60 ist drehbar in der Hülse 54 gelagert. Es ist um eine radiale Achse drehbar. Es bildet an seiner Unterkante ein Werkzeug, z.B. eine Rippe 64, die auch als Blatt oder Klinge beschrieben werden kann. Die schmale Rippe 64 ragt in den freien Hohlraum der Hülse 54 vor, wie auch aus Figur 6 ersichtlich ist.

Beim Zusammenstecken im Sinne des Pfeils 62 gelangt die Rippe 64 in eine Rinne 66 im Ventilkörper 46 und anschließend in den Betätigungsschlitz 44 des ersten Teils 58. Im vollständig zusammengesteckten Zustand ist sie nicht mehr in der Rinne 66, sondern nur noch im Betätigungsschlitz 44. Wird nun das zweite Teil 60 gedreht, dreht sich auch das erste Teil 58 mit und dementsprechend auch der Ventilkörper 46.

In der in den Figuren 3 und 5 gezeigten Stellung befindet sich der Ventilkörper 46 im Schließzustand, die Passage durch das Ventilgehäuse 36 ist vollständig gesperrt. In diesem Zustand ist es möglich, den Trichteradapter 32 vom Ventil 34 in Gegenrichtung zum Pfeil 62 abzuziehen. In jeder anderen Drehposition, auch in einer Zwischenstellung, ist dies nicht möglich.

Wenn ein Patient das erste Teil 58 oder das zweite Teil 60 ausgehend von der vorgenannten Stellung verdreht, ist ein Zusammenstecken im Sinne des Pfeils 62 nicht möglich. Dieses ist erst dann möglich, wenn die beiden Teile 58, 60 sich in dem Zustand befinden, in dem sie in den Figuren 3 bis 6 gezeigt sind.

In den Figuren 4 und 6 ist ein Griffteil des zweiten Teils 60 in Axialrichtung ausgerichtet dargestellt. Dieses Griffteil sollte besser um 90° um die Drehachse des zweiten Teils 60 verdreht angeordnet werden, damit es unmittelbar den Zustand des Ventilkörpers 46 anzeigt. In Querstellung des Griffteils ist auch das Ventil geschlossen. In Längsstellung, wie in Figur 4 gezeigt, aber im Unterschied zu dieser Figur, ist dann das Ventil 34 geöffnet.

Andere Ausbildungen sind möglich. So kann beispielsweise ein Werkzeug in Form eines Mehrkants die Rippe 64 ersetzen, der stiftförmig vorspringt. Die Rinne 66 kann ansteigend ausgebildet werden, so dass der Mehrkant angehoben wird und auf die Oberseite des ersten Teils 58 gerät. Beim weiteren Zusammenschieben fällt er dann in eine Ausnehmung hinein, die dem Mehrkant entspricht und die den Betätigungsschlitz 44 ersetzt. Beim Lösen muss das zweite Teil 60 hochgezogen werden.

Figur 7 schließlich zeigt ein Ausführungsbeispiel, bei dem das Zusammenstecken von Trichteradapter 32 und Ventil 34 und das Zusammenfinden von erstem Teil 58 und zweitem Teil 60 der Handhabe 42 nicht gleichzeitig erfolgt und unabhängig voneinander ist. Das zweite Teil 60 ist nun an einer biegsamen Lasche 68 angeordnet, die mit dem Trichteradapter 32 fest verbunden ist. Am freien Ende der Lasche ist das zweite Teil 60 angebracht. Es hat einen Griffbereich und auf der anderen Seite der Lasche ein Werkzeug, das der Rippe 64 entspricht, beispielsweise in Form eines Werkzeugs bzw. Schlüssels beliebiger Form. Im ersten Teil 58 ist eine passgenaue Ausnehmung 70 vorgesehen, die den Betätigungsschlitz 44 ersetzt. In diese Ausnehmung 70 wird das Werkzeug hineingedrückt, danach kann über den Griffbereich der Ventilkörper 46 betätigt werden.

Die PEG-Sonde hat einen PEG-Schlauch 22, eine äußere Halteplatte 30, durch die der PEG-Schlauch 22 geführt und in der er fixiert ist, und einen Trichteradapter 32, der einem äußeren Endbereich 26 des PEG-Schlauchs 22 zugeordnet ist. Ein dicht schließendes Ventil 34 ist entweder im PEG-Schlauch 22 zwischen der äußeren Halteplatte 30 und dem äußeren Endbereich 26 oder am äußeren Endbereich 26 des PEG-Schlauchs 22 angeordnet. Das Ventil 34 sperrt in einer Schließstellung den Durchfluss durch den PEG-Schlauch 22 und gibt in einer Öffnungsstellung zumindest im vollen Querschnitt des PEG-Schlauchs 22 frei. Das Ventil 34 weist einen Ventilkörper 46 und ein Ventilgehäuse 36 auf. Der Ventilkörper 46 ist mit einer von außen zugänglichen Handhabe 42 verbunden und bewegbar, er ist im Ventilgehäuse 36 gelagert. Vorzugsweise hat die Handhabe 42 einen Betätigungsschlitz oder einen entsprechenden Vorsprung bzw. eine entsprechende Vertiefung, die eine Betätigung durch ein passendes, separates Werkzeug, nicht aber durch die Finger eines Menschen oder ein einfaches Hilfsmittel zulassen.

## Patentansprüche

1. PEG-Sonde
- mit einer inneren Halteplatte,
- mit einem PEG-Schlauch (22), der einen inneren Endbereich und einen äußeren Endbereich (26) aufweist, wobei der innere Endbereich (24) mit der inneren Halteplatte (20) fest verbunden ist und dort eine innere Öffnung (28) des PEG-Schlauchs (22) aufweist,
- mit einer äußeren Halteplatte (30), durch die der PEG-Schlauch (22) geführt und in der er fixiert ist,
- mit einem Trichteradapter (32), der dem äußeren Endbereich (26) zugeordnet ist,
- mit einem dicht schließenden Ventil (34), das am äußeren Endbereich (26) des PEG-Schlauchs (22) angeordnet ist, wobei das Ventil (34) in einer Schließstellung den Durchfluss durch den PEG-Schlauch (22) sperrt, in einer Zwischenstellung teilweise freigibt und in einer Öffnungsstellung zumindest im vollen Querschnitt des PEG-Schlauchs (22) freigibt, wobei das Ventil (34) einen Ventilkörper (46) und ein Ventilgehäuse (36) aufweist, der Ventilkörper (46) mit einer von außen zugänglichen Handhabe (42) verbunden und zwischen der dauerhaften Schließstellung und der dauerhaften Öffnungsstellung bewegbar in dem Ventilgehäuse (36) gelagert ist, wobei die Handhabe (42) mehrteilig ausgebildet ist, ein erstes Teil der Handhabe (58) permanent am Ventilkörper (46) angeordnet ist, und ein zweites Teil der Handhabe (60) separat vom Ventil (34) am Trichteradapter (32) angeordnet ist und beim Zusammenstecken von Ventil (34) und Trichteradapter (32) selbsttätig in das erste Teil der Handhabe (58) eingreift, und das zweite Teil der Handhabe (60) ein Werkzeug aufweist, das mit einem angepassten Gegenwerkzeug des ersten Teils zusammenwirkt, wobei der PEG-Schlauch (22) sich nur auf einer inneren Seite des Ventils (36) befindet, das Ventil (34) an der inneren Seite mit dem Endbereich des PEG-Schlauchs (22) verbunden ist und an seiner anderen, äußeren Seite ein Kupplungsstück (50) aufweist, der Trichteradapter (32) ein Kupplungsgegenstück (52) aufweist, das dem Kupplungsstück (50) angepasst ist, und dass Kupplungsstück (50) und Kupplungsgegenstück (52) dicht miteinander mechanisch verbindbar und lösbar sind.

2. PEG-Sonde nach Anspruch 1, **dadurch gekennzeichnet, dass** das Werkzeug, das das zweite Teil der Handhabe (60) aufweist, mit einem angepassten Gegenwerkzeug des ersten Teils formschlüssig verbindbar ist.

3. PEG-Sonde nach einem der vorangegangenen Ansprüche, **dadurch gekennzeichnet, dass** das Ventilgehäuse (36) zweiteilig ausgebildet ist, dass die beiden Teilstücke des Ventilgehäuses (36) lösbar miteinander verbindbar sind.

4. PEG-Sonde nach dem vorangegangenen Anspruch, **dadurch gekennzeichnet, dass** sich der Ventilkörper (46) zwischen den beiden Teilstücken des Ventilgehäuses (36) befindet, dass der Ventilkörper (46) im Ventilgehäuse (36) festgehalten ist, wenn die beiden Teilstücke des Ventilgehäuses (36) verbunden sind, und dass der Ventilkörper (46) vom Ventilgehäuse (36) freikommt, wenn die beiden Teilstücke des Ventilgehäuses (36) voneinander getrennt werden.

5. PEG-Sonde nach einem der vorangegangenen Ansprüche, **dadurch gekennzeichnet, dass** der Ventilkörper (46) kugelförmig, kegelförmig, zylindrisch oder scheibenförmig ist.

6. PEG-Sonde nach einem der vorangegangenen Ansprüche, **dadurch gekennzeichnet, dass** das zweite Teil der Handhabe (60) starr am Trichteradapter (32) angeordnet ist.

7. PEG-Sonde nach Anspruch 1, **dadurch gekennzeichnet, dass** das zweite Teil der Handhabe (60) flexibel mit dem Trichteradapter (32) verbunden ist.

8. PEG-Sonde nach Anspruch 1, **dadurch gekennzeichnet, dass** das Ventil (34) eine Rille oder Rippe aufweist, die ein Werkzeug des zweiten Teils beim Zusammenfügen von Trichteradapter (32) und Ventil (34) aufnimmt und führt.

## Claims

1. A PEG tube
- with an inner retaining plate,
- with a PEG hose (22) having an inner end portion and an outer end portion (26), the inner end portion (24) being firmly connected to the inner retaining plate (20) and having an inner opening (28) of the PEG hose (22) there,
- with an outer retaining plate (30) through which the PEG hose (22) is routed and in which it is fixed,
- with a funnel adapter (32) associated with the outer end portion (26),
- with a tightly sealing valve (34) disposed at the outer end portion (26) of the PEG hose (22), wherein the valve (34) blocks the flow-through through the PEG hose (22) in a closing position, partially unblocks it in an intermediate position, and unblocks it at least over the full cross section of the PEG hose (22) in an opening position, wherein the valve (34) comprises a valve body (46) and a valve housing (36), the valve body (46) is connected to a handling means (42) accessible from the outside and is mounted in the valve housing (36) so as to be movable between the permanent closing position and the permanent opening position, wherein the handling means (42) has a multi-part configuration, a first part of the handling means (58) is permanently disposed on the valve body (46) and a second part of the handling means (60) is disposed on the funnel adapter (32) separately from the valve (34) and, when the valve (34) and the funnel adapter (32) are plugged together, automatically engages the first part of the handling means (58), and the second part of the handling means (60) comprises a tool cooperating with a matching tool counterpart of the first part, wherein the PEG hose (22) is located only on an inner side of the valve (36), the valve (34) is connected on the inner side to the end portion of the PEG hose (22) and has at its other, outer side a coupling portion (50), the funnel adapter (32) has a coupling counterpart (52) matched to the coupling portion (50), and that the coupling portion (50) and the coupling counterpart (52) can be mechanically connected to and detached from each other in a tight manner.

2. The PEG tube according to claim 1, **characterized in that** the tool, which the second part of the handling means (60) comprises, can be positively connected to a matching tool counterpart of the first part.

3. The PEG tube according to any one of the preceding claims, **characterized in that** the valve housing (36) is configured in two parts, that the two sections of the valve housing (36) can be connected to each other in a detachable manner.

4. The PEG tube according to the preceding claim, **characterized in that** the valve body (46) is located between the two sections of the valve housing (36), that the valve body (46) is retained in the valve housing (36) when the two sections of the valve housing (36) are connected, and that the valve body (46) comes free from the valve housing (36) when the two sections of the valve housing (36) are separated from one another.

5. The PEG tube according to any one of the preceding claims, **characterized in that** the valve body (46) is spherical, conical, cylindrical or disk-shaped.

6. The PEG tube according to any one of the preceding claims, **characterized in that** the second part of the handling means (60) is rigidly disposed on the funnel adapter (32).

7. The PEG tube according to claim 1, **characterized in that** the second part of the handling means (60) is flexibly connected to the funnel adapter (32).

8. The PEG tube according to claim 1, **characterized in that** the valve (34) comprises a groove or a rib that receives and guides a tool of the second part during the joining of the funnel adapter (32) and the valve (34).

## Revendications

1. Sonde GPE comprenant
- une plaque de retenue intérieure,
- un tuyau flexible GPE (22) qui présente une zone d'extrémité intérieure et une zone d'extrémité extérieure (26), dans lequel ladite zone d'extrémité intérieure (24) est solidarisée à la plaque de retenue intérieure (20) et y présente un orifice intérieur (28) du tuyau flexible GPE (22),
- une plaque de retenue extérieure (30) qui est traversée par ledit tuyau flexible GPE (22) et dans laquelle celui-ci est fixé,
- un adaptateur à entonnoir (32) qui est associé à ladite zone d'extrémité extérieure (26),
- une soupape (34) à fermeture étanche qui est disposée à la zone d'extrémité extérieure (26) du tuyau flexible GPE (22), dans laquelle ladite soupape (34), une fois dans une position de fermeture, bloque le passage à travers le tuyau flexible GPE (22), libère celui-ci en partie dans une position intermédiaire, et, une fois dans une position d'ouverture, le libère au moins dans toute la section transversale du tuyau flexible GPE (22), dans laquelle la soupape (34) comprend un corps de soupape (46) et un boîtier de soupape (36), ledit corps de soupape (46) est relié à une manette (42) accessible de l'extérieur et est logé dans ledit boîtier de soupape (36) de manière déplaçable entre la position de fermeture durable et la position d'ouverture durable, dans laquelle ladite manette (42) est réalisée en plusieurs parties, une première partie de la manette (58) est disposée de manière permanente sur le corps de soupape (46) et une deuxième partie de la manette (60) est disposée sur ledit adaptateur à entonnoir (32) de manière distincte de la soupape (34) et s'engage automatiquement dans la première partie de la manette (58) lorsque la soupape (34) et l'adaptateur à entonnoir (32) sont emboîtés, et ladite deuxième partie de la manette (60) comprend un outil qui agit de concert avec un contre-outil adapté de la première partie, dans laquelle le tuyau flexible GPE (22) ne se trouve que sur une face intérieure de la soupape (34), la soupape (34) est reliée, sur la face intérieure, à la zone d'extrémité du tuyau flexible GPE (22) et comprend, sur son autre face extérieure, une pièce de couplage (50), l'adaptateur à entonnoir (32) présente une contre-pièce de couplage (52) qui est adaptée à la pièce de couplage (50), et que la pièce de couplage (50) et la contre-pièce de couplage (52) peuvent être reliées mécaniquement entre elles de manière étanche et être détachées.

2. Sonde GPE selon la revendication 1, **caractérisée par le fait que** l'outil que présente la deuxième partie de la manette (60) peut être relié à engagement positif à un contre-outil adapté de la première partie.

3. Sonde GPE selon l'une quelconque des revendications précédentes, **caractérisée par le fait que** le boîtier de soupape (36) est réalisé en deux parties, que les deux parties du boîtier de soupape (36) peuvent être reliées entre elles de manière amovible.

4. Sonde GPE selon la revendication précédente, **caractérisée par le fait que** le corps de soupape (46) est situé entre les deux parties du boîtier de soupape (36), que le corps de soupape (46) est retenu à l'intérieur du boîtier de soupape (36) lorsque les deux parties du boîtier de soupape (36) sont reliées entre elles, et que le corps de soupape (46) est dégagé du boîtier de soupape (36) lorsque les deux parties du boîtier de soupape (36) sont séparées l'une de l'autre.

5. Sonde GPE selon l'une quelconque des revendications précédentes, **caractérisée par le fait que** le corps de soupape (46) est sphérique, conique, cylindrique ou en forme de disque.

6. Sonde GPE selon l'une quelconque des revendications précédentes, **caractérisée par le fait que** la deuxième partie de la manette (60) est disposée rigidement sur ledit adaptateur à entonnoir (32).

7. Sonde GPE selon la revendication 1, **caractérisée par le fait que** la deuxième partie de la manette (60) est reliée de manière flexible à l'adaptateur à entonnoir (32).

8. Sonde GPE selon la revendication 1, **caractérisée par le fait que** la soupape (34) présente une cannelure ou nervure qui loge et guide un outil de la deuxième partie lorsque l'adaptateur à entonnoir (32) et la soupape (34) sont assemblés.
